# EUROPEAN PATENT APPLICATION

(11) **EP 2 108 958 A1**
(43) Date of publication of application: **14.10.2009**
(21) Application number: 08154267.2
(22) Date of filing: 09.04.2008
(51) Int. Cl.: G01N 33/68

(54) **Pro-Endothelin-1 for the prediction of impaired peak oxygen consumption**

(71) Applicant: B.R.A.H.M.S. Aktiengesellschaft, 16761 Hennigsdorf (DE)
(72) Inventor: Struck, Joachim, 13465 Berlin (DE); Morgenthaler, Nils, 13503 Berlin (DE); Bergmann, Andreas, 12351 Berlin (DE); Müller, Christian, 4031 Basel (CH)
(74) Representative: Kilger, Ute

(57) **Abstract**

Subject of the present invention is an in vitro method for the alternative assessment of peak oxygen consumption (VO₂) for a subject not having a heart failure by measuring Pro-Endothelin-1 (ProET-1) or fragments thereof.

## Description

Subject of the present invention is an *in vitro* method for the prediction of impaired peak oxygen consumption (VO₂) for a subject not having a heart failure:
- determining the level of Pro-Endothelin-1 (ProET-1) or fragments thereof of at least 12 amino acids in a sample obtained from said subject;
- using said level of Pro-Endothelin-1 (ProET-1) or fragments thereof of at least 12 amino acids as a surrogate marker for peak VO₂.

Another subject of the invention is the use of this method for risk assessment before non-cardiac surgery.

The clinical use of maximal cardiopulmonary exercise (CPET) with measurement of peak VO₂ is well established (Cappuccio et al, Eur J Clin Invest 1991;21:40-6; Benzo et al, Respir Med 2007;101:1790-7; Bolliger et al, Am J Respir Crit Care Med 1995;151:1472-80; Brutsche et al, Eur Respir J 2000;15:828-32; Butler et al, J Am Coll Cardiol 2004;43:787-93; Isnard et al, Am J Cardiol 2000;86:417-21; Isnard et al, Am Heart J 2003;146:729-35). However, due to required equipment, time as well as a high degree of knowledge by both the technician and physician, CPET is not a broadly applied technique. Notably, estimation of peak VO₂ based on the maximal workload achieved during a standard exercise test without gas exchange techniques, i.e, speed and grade of the treadmill or resistance of the bicycle ergometer, may be associated with significant inaccuracies, particularly in patients with low exercise capacity (Benzo et al, Chest 2007;132:1500-5, Myers et al, J Am Coll Cardiol 1991;17:1334-42), and is an insufficient alternative to CPET. Thus, rapidly and easily available alternatives to the assessment of peak VO₂ would be highly desirable. In a previous study among patients with chronic heart failure (HF), B-type natriuretic peptide (BNP) has been proposed as a biochemical tool for the differentiation of patients with severely and moderately impaired exercise capacity (Kruger et al, J Am Coll Cardiol 2002;40:718-22). (BNP is derived from a larger precursor peptide, proBNP, which can be proteolytically cleaved into BNP and N-tenninal proBNP (NT-proBNP)). However, other studies in HF patients revealed that the correlation between BNP and peak VO₂ might not be strong enough for reliable assessment of peak VO₂ (Brunner-La Rocca et al, Heart 1999;81:121-7, Maeder et al, Int J Cardiol 2007;120:391-8). Furthermore, estimation of exercise capacity is required not only in HF patients. Similar studies for less selected patient groups and studies with other biomarkers are not available.

Thus, it was the objective of the present invention to find another marker as a surrogate for the assessment of peak VO₂ by CPET. A surrogate marker according to the present invention is a substitute for the direct assessment of peak VO₂ by CPET.

The marker assessed in the present invention is related to Endothelin-1. Endothelin (ET)-1 is a potent endothelium-derived endogenous vasoconstrictor (13). ET-1 exerts its vascular effects by activation of ET(A) and ET(B) receptors on smooth muscle cells, which causes an increase in intracellular calcium (Yanagisawa et al, J Hypertens Suppl 1988;6:S188-91). Endogenous ET-1 contributes significantly to the vascular tone at rest (Spratt et al, Br J Pharmacol 2001;134:648-54), and ET(A) receptor-mediated vasoconstriction has been shown to account at least in part for the impaired vasodilatory response to exercise in hypertensive subjects (McEniery et al, Hypertension 2002;40:202-6). Elevated ET-1 levels have been found in conditions associated with impaired exercise capacity, including post-myocardial infarction (Omland et al, Circulation 1994;89:1573-9) and HF (Wei et al, Circulation 1994;89:1580-6), but also chronic obstructive pulmonary disease (COPD) (Ferri et al, J Clin Pathol 1995;48:519-24). In HF patients, a correlation between ET-1 and peak VO₂ has been suggested previously (Kinugawa et al, J Card Fail 2003;9:318-24). HF patients seem to exhibit an upregulation of neurohormones including ET-1 already without exercise testing.

Object of the present invention was the provision of a marker of peak VO₂ in unselected patients, especially in subjects not having heart failure.

Thus, subject of the present invention is an *in vitro* method for the prediction of impaired peak VO₂ (<14 ml/kg/min) for a subject not having heart failure:
- determining the level of Pro-Endothelin-1 (ProET-1) or fragments thereof of at least 12 amino acids in a sample obtained from said subject;
- using said level of Pro-Endothelin-1 (ProET-1) or fragments thereof of at least 12 amino acids as a surrogate marker for peak VO₂.

In a preferred embodiment of the method according to the invention the sample was taken from said subject at rest and thus resting levels of Pro-Endothelin-1 (ProET-1) or fragments thereof of at least 12 amino acids are determined.

In another preferred embodiment of the method according to the invention the level of Pro-Endothelin-1 (ProET-1) or fragments thereof of at least 12 amino acids is additionally measured to maximal body weight-indexed bicycle workload (WLₘₐₓ) and wherein the measurement of Pro-Endothelin-1 (ProET-1) or fragments thereof of at least 12 amino acids leads to an improvement in the accuracy of WLₘₐₓ for the prediction of impaired peak VO₂ (<14 ml/kg/min). WLₘₐₓ is the maximal workload achieved during CEPT divided by the individual's body weight [Watt/kg].

VO₂ max or peak VO₂ (also maximal oxygen consumption, maximal oxygen uptake or aerobic capacity) is the maximum capacity of an individual's body to transport and utilize oxygen during incremental exercise, which reflects the physical fitness of the individual. VO₂ max is properly defined by the Fick Equation: VO₂ max = Q(CaO₂ - CvO₂), wherein Q is the cardiac output of the heart, CaO₂ is the arterial oxygen content, and CvO₂ is the venous oxygen content. In general clinical and athletic testing, the measuring of VO₂ max usually involves a graded exercise test (either on a treadmill or on a cycle ergometer) in which exercise intensity is progressively increased while measuring ventilation and oxygen and carbon dioxide concentration of the inhaled and exhaled air. VO₂ max is reached when oxygen consumption remains at steady state despite an increase in workload. VO₂ max is widely accepted as a good measure of cardiovascular fitness and maximal aerobic power.

A surrogate marker according to the present invention is a substitute for the direct assessment of peak VO₂ by CPET.

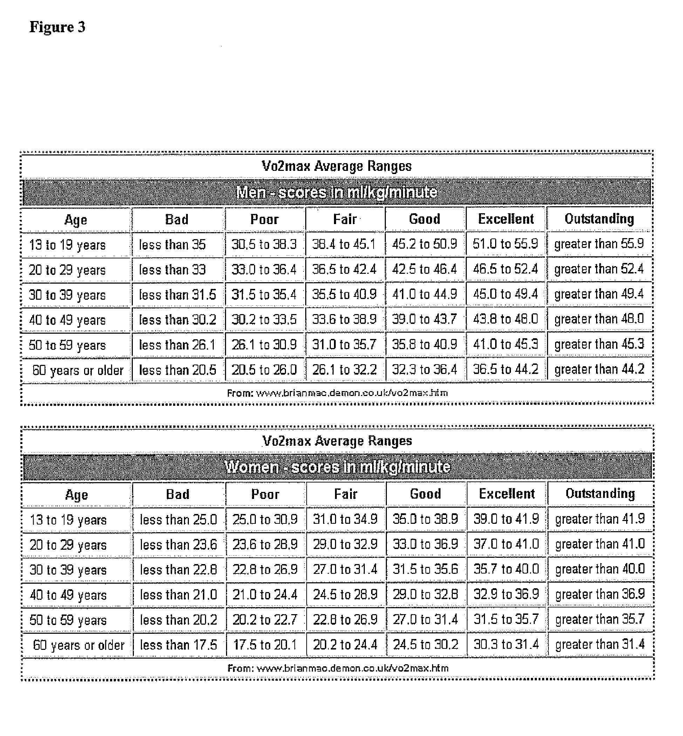

The appraisal of peak VO₂ (VO₂ max) has to take into consideration the individual's gender and age. A peak VO₂ of 14 (ml/kg/min) or less can be considered as severely reduced, since frequently it qualifies for a heart transplantation (http://www.chfpatients.com/tests/vo2.htm), see Figure 3.

Endothelin-1 is derived from a larger precursor molecule named Pro-Endothclin-1. Pro-Endothelin-1 can be proteolytically processed into various fragments as described (Struck J, Morgenthaler NG, Bergmann A. Proteolytic processing pattern of the endothelin-1 precursor in vivo. Peptides. 2005 Dec;26(12):2482-6.). These fragments are subject to proteolytic degradation in the blood circulation, which can happen quickly or slowly, depending on the type of fragment and the type and concentration/activity of proteases present in the circulation. Thus, according to the present invention the level of any of these fragments of at least 12 amino acids may be measured, preferably fragments of at least 20 amino acids, more preferably of at least 30 amino acids. Preferably, C-terminal pro-ET-1 (CT-proET-1) or a fragment thereof may be measured.

In another preferred embodiment of the invention the level of proBNP or fragments thereof of at least 12 amino acids including BNP or NT-proBNP is measured in addition to WLₘₐₓ and wherein the measurement of proBNP or fragments thereof of at least 12 amino acids leads to an improvement in the accuracy of WLₘₐₓ for the prediction of impaired peak VO₂. Thus, according to the present invention the level of any of these fragments of at least 12 amino acids may be measured, preferably fragments of at least 20 amino acids, more preferably of at least 30 amino acids.

In an especially preferred embodiment both the level of Pro-Endothelin-1 (ProET-1) or fragments thereof of at least 12 amino acids and proBNP or fragments thereof of at least 12 amino acids including BNP or NT-proBNP is measured in addition to WLₘₐₓ and wherein the measurement of Pro-Endothelin-1 (ProET-1) or fragments thereof of at least 12 amino acids and proBNP or fragments thereof of at least 12 amino acids including BNP or NT-proBNP leads to an improvement in the accuracy of WLₘₐₓ for the prediction of impaired peak VO₂.

When CT-proET-1 and BNP are used as fragments of their precursors, preferable cut-off values are 74.4 pmol/L and 35.9 pg/mL, respectively. Thus, the use of these two biomarkers in combination with a standard exercise test for the prediction of a severely impaired peak VO₂ is subject of the present invention.

Subject of the present invention is further the use of Pro-Endothelin-1, preferably CT-proET-1, in clinical practice in situations where a semi-quantitative estimate of peak VO₂ seems sufficient, e.g., for risk assessment before non-cardiac surgery. For low- or medium-risk surgery in patients without significant risk factors such as angina, symptomatic HF, or diabetes, guidelines suggest an exercise capacity threshold of four metabolic equivalents as the criterion to decide about the need for additional non-invasive tests (Eagle et al, Circulation 2002;105:1257-67).

Thus, subject of the present invention is the use of CT-proET-1 for preoperative risk assessment. One might also consider a combination of BNP and CT-proET-1.

According to the present invention said sample that is obtained from a subject not having heart failure is selected from the group comprising a blood sample, a serum sample, and a plasma sample.

According to a preferred embodiment of the invention said level of CT-proET-1 (or fragments thereof) is measured with a sandwich immunoassay. An example for such sandwich immunoassay has been described (Papassotiriou J, Morgenthaler NG, Struck J, Alonso C, Bergmann A. Immunoluminometric assay for measurement of the C-terminal endothelin-1 precursor fragment in human plasma. Clin Chem. 2006 Jun; 52(6): 1144-51.)

Instead of Pro-Endothelin-1 (ProET-1) or fragments thereof another surrogate marker may be used selected from the group comprising Pro-Adrenomedullin or fragments thereof of at least 12 amino acids including MR-proADM, CT-proADM, Adrenomedullin and PAMP (DE 103 16 583.5; DE 10 2006 060 11; Beltowski J, Jamroz A. Adrenomedullin--what do we know 10 years since its discovery? Pol J Pharmacol. 2004 Jan-Feb;56(1):5-27.; Struck J, Tao C, Morgenthaler NG, Bergmann A. Identification of an Adrenomedullin precursor fragment in plasma of sepsis patients. Peptides. 2004 Aug;25(8):1369-72. ).

### Figure Description

### Figure 1:

Title: Relationship between peak oxygen consumption, external workload and biomarkers Caption: Scatter plots illustrating the correlations between peak oxygen consumption (peak VO₂) and B-type natriuretic peptide (BNP, panel A), C-terminal-pro-endothelin-1 (CT-proET-1, panel B), and the maximal body weight-indexed external bicycle workload (WLₘₐₓ, panel C) and as well as the correlation between BNP and CT-proET-1 (panel D). Spearman correlation coefficients are given. Note the logarithmic scale for peak VO₂ and BNP.

### Figure 2:

Title: Accuracy of biomarkers alone or combined with external workload for the prediction of a peak oxygen consumption <14 ml/kg/min.

Caption: Panel A: Receiver-operator-characteristics (ROC) curves showing the AUC (with 95% confidences intervals) for the prediction of a peak VO₂ <14 ml/kg/min for B-type natriuretic peptide (BNP) and C-terminal-pro-endothelin-1 (CT-proET-1) at rest. Panel B: ROC curves showing the AUC (with 95% confidences intervals) for the prediction of a peak VO₂ <14 ml/kg/min for BNP and CT-proET-1 at rest and at peak exercise. Panel C: ROC curves showing the AUC for the prediction of a peak VO₂ <14 ml/kg/min for BNP and CT-proET-1 at rest and the maximal body weight-indexed external bicycle workload (WLₘₐₓ). For better comparison with BNP and CT-proET-1 the ROC curve for 1/WLₘₐₓ is shown. In addition, sensitivity and 1-specificity of the combinations of optimal cut-offs for WLₘₐₓ with optimal cut-offs for CT-proET-1 and BNP are shown. The arrows indicate sensitivity and 1-specificity of the optimal WLₘₐₓ cut-off (→) and combinations of WLₘₐₓ, CT-proET-1, and BNP to achieve optimal specificity (►) or sensitivity (■■►).

### Figure 3:

VO₂ max average ranges for women and men.

### Examples

### Summary

The present study of a representative sample of unselected patients referred for CPET revealed two important findings: First, CT-proET-1 measured at rest was related to peak VO₂ and had a high accuracy in predicting a severely impaired peak VO₂ and thereby outperformed BNP. Second, additional measurement of CT-proET-1 and BNP led to a marked improvement in the accuracy of WLₘₐₓ for the prediction of severely impaired peak VO₂.

### Methods

### Study population

From May 2004 to June 2005, 185 consecutive patients referred for CPET for the evaluation of unexplained exercise intolerance were eligible for inclusion. We excluded patients without informed consent (n=12) and patients without CT-pro-ET-1 and/or BNP determination (n=11). This left a total of 162 patients for the present analysis. This study was approved by the local ethics committee. Written informed consent was obtained from all participating patients.

### CPET procedures

Patients underwent symptom-limited upright bicycle exercise using routine protocols with a 12-lead electrocardiogram recording each minute of exercise and continuous monitoring of the electrocardiogram throughout the test. The following test end points were applied: physical exhaustion, severe angina, sustained ventricular arrhythmia or exertional hypotension. Expired gases were acquired continuously, and oxygen uptake (VO₂) and carbon dioxide output (VCO₂) were recorded in rolling 30 second averages. Calibration of the system was performed before each test with a 3-liter syringe. The respiratory exchange ratio respiratory exchange ration (RER), defined as VCO₂ divided by VO₂, was determined throughout the test. Maximal effort was assumed if at least one of the following criteria was fulfilled: 1. peak heart rate >80% predicted, 2. RER >1.2, 3. lactate at peak exercise >4.5 mmol/l, or 4. base excess >-2.5 mmol/l (Schmid et al, Respirology 2007;12:916-23). These criteria have been used in our CPET laboratory for years and reflect the principles outlined in the guidelines of the American Thorax Society/Amercian College of Chest Physicians (ATS/ACCP Statement on cardiopulmonary exercise testing, Am J Respir Crit Care Med 2003;167:211-77). Exercise capacity was expressed as directly measured peak VO₂ and the maximal body weight-indexed external bicycle workload (WLₘₐₓ).

### Blood sampling and laboratory methods

A specimen of venous blood was drawn before and one minute after peak exercise in the seated position from a catheter previously inserted into the antecubital vein. These samples were collected in plastic tubes containing ethylene-diamin-tetra-acetate. They were placed on ice and then centrifuged at 3,000 g; and plasma was frozen at -80°C, All plasma samples were analyzed approximately one year after collection. The laboratory technician who performed the assays was at a different site and blinded to patients characteristics and CPET results. CT-proET-1 was detected using a novel commercially available assay in the chemiluminescence/coated tube-format (BRAHMS AG, Hennigsdorf/Berlin, Germany) as previously described (Papassotiriou et al, Clin Chem 2006;52:1144-51; Khan et al, Am Heart J 2007;154:736-42). BNP concentration was determined using the commercially available AxSYM BNP assay (Abbott Laboratories, Zug, Switzerland) (Mueller et al, Clin Chem 2004;50:1104-6). This fully automated microparticle enzyme immunoassay uses two monoclonal mouse antibodies in a two-step format. Being harmonized to the Biosite BNP assay, it has a dynamic range of 1-4000 pg/ml.

### Statistical analysis

Data were expressed as numbers and percentages, mean±standard deviation, and median (interquartile range) unless otherwise indicated. Normal distribution of the data was tested with the use of the Kolmogorov-Smimov test. Comparisons were made using chi-square or Fisher exact tests, unpaired t-tests, or nonparametric tests as appropriate. To compare BNP (log-transformed due to skewed distribution) and CT-proET-1 levels at rest and at peak exercise analysis of variance for repeated measures was performed. Correlations between parameters of interest were performed using Spearman correlation coefficients. ROC curves were constructed to assess the sensitivity and specificity of CT-proET-1 and BNP throughout the concentrations and WLₘₐₓ to detect a peak VO₂ <14 ml/kg/min. This cut-off was selected based on its established prognostic value in patients with HF (Cappuccio et al, Eur J Clin Invest 1991;21:40-6) and the fact that it corresponds to four metabolic equivalents, which is a frequently used cut-off for the differentiation of severely and moderately impaired exercise capacity for preoperative risk stratification (Eagle et al, Circulation 2002;105:1257-67). Sensitivity, specificity, positive predictive value, and negative predictive values for optimal cut-offs of biomarkers and WLₘₐₓ as well as their combination were calculated. ROC curves were compared using the method by DeLong et al. (DeLong et al, Biometrics 1988;44:837-45) using a specialized software (Analyse-it V2.04, Leeds, UK). All other statistical analyses were performed using SPSS/PC (version 15.0, SPSS Inc, Chicago, IL). All hypothesis testing was two-tailed. A p value <0.05 was considered statistically significant.

### Results

### Patients

Characteristics of patients achieving a peak VO₂ <14 ml/kg/min (n=39) versus ≥14 ml/kg/min (n=123) are presented in Table 1. Patients with a peak VO₂ <14 ml/kg/min were older, more overweight, were more likely to have coronary artery disease, peripheral arterial obstructive disease, chronic obstructive pulmonary disease, and pulmonary hypertension, were more likely to be treated with beta blockers, inhibitors of the renin-angiotensin-aldosterone system, diuretics, nitrates, aspirin, and statins, and were more symptomatic than patients with peak VO₂ ≥ 14 ml/kg/min.

### CPET

Results from CPET are shown in Table 2. Peak heart rate, peak systolic blood pressure, and per definition, measures of exercise capacity were lower in patients with peak VO₂ <14 ml/kg/min than in those with peak VO₂ ≥14 ml/kg/min. The forced expiratory volume within the first second and the ratio of the forced expiratory volume within the first second to forced vital capacity was lower, and the resting respiratory rate was higher in patients with a peak VO₂ <14 ml/kg/min. The arterial pressure of oxygen at peak exercise but not at rest and the arterial carbon dioxide pressure at rest but not at peak exercise were lower, and the alveolo-arterial gradient both at rest and at peak exercise was higher in patients with a peak VO₂ <14 ml/kg/min as compared to those with a peak VO₂ ≥14 ml/kg/min.

### CT-proET-1 and BNP kinetics

In Table 3, biomarker levels at rest and at peak exercise as well as absolute and relative changes from rest to peak exercise in patients with a peak VO₂ <14 ml/kg/min versus ≥14 ml/kg/min are displayed. CT-proET-1 and BNP levels at rest (p<0.001 for both) and at peak exercise (p<0.001 for both) were higher in patients with a peak VO₂ <14 ml/kg/min as compared to those with a peak VO₂ ≥14 ml/kg/min. There was a significant increase in CT-pro-ET-1 and BNP from rest to peak exercise in both patients with peak VO₂ <14 ml/kg/min and ≥14 ml/kg/min. Absolute and relative changes in CT-proET-1 and BNP were similar in both groups.

### Relationship between CT-proET-1, BNP, and WLₘₐₓ and peak VO₂

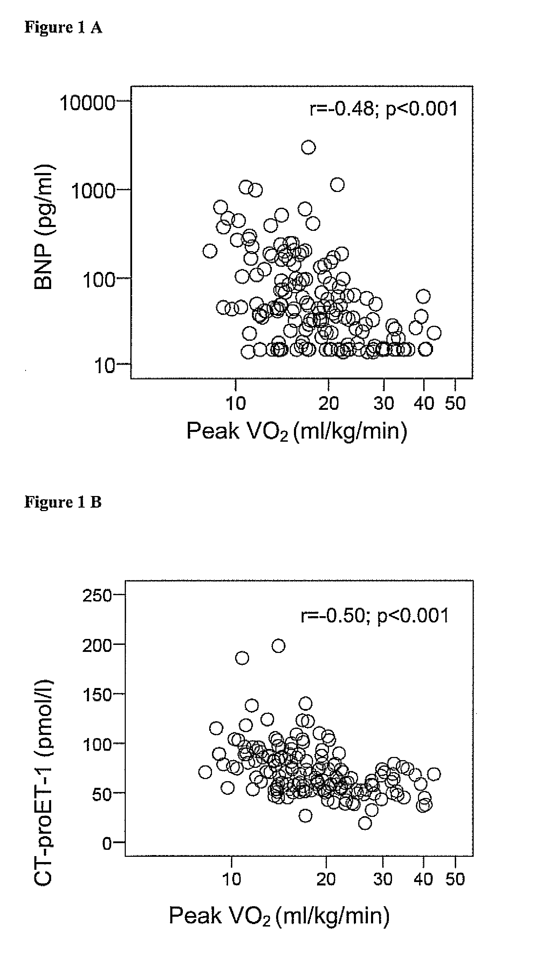

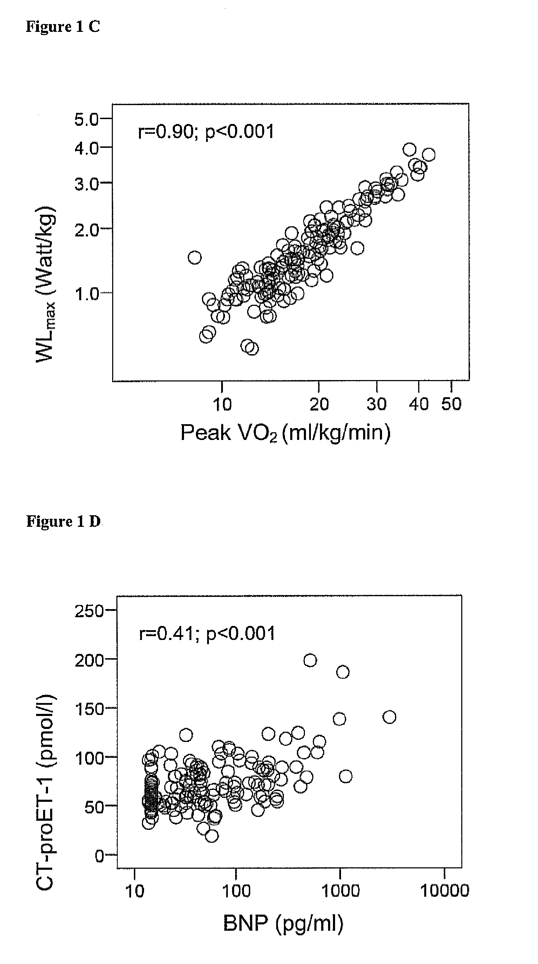

As shown in Figure 1, there were statistically significant but only moderate correlations between CT-proET-1 and BNP and peak VO₂ (panels A and B). In contrast, WLₘₐₓ and peak VO₂ were closely related (panel C). There was also a moderate correlation between CT-proET-1 and BNP (panel D).

### CT-proET- and BNP at rest for the prediction of a peak VO₂ <14 ml/kg/min

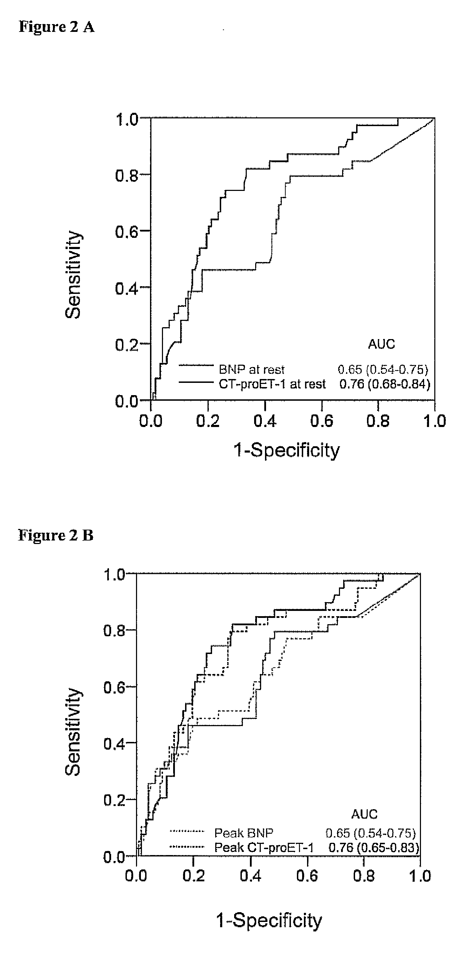

In Figure 2A, the ROC curves for resting CT-proET-1 and BNP levels for the prediction of a peak VO₂ <14 ml/kg/min are displayed. The area under the curve (AUC) for CT-proET-1 was 0.76 [95%confidence interval (95%CI) 0.68-0.84; p<0.001], and the AUC for BNP was 0.65 (95%Cl 0.54-0.75; p=0.006). Comparison of the curves revealed a significant difference in favor of CT-proET-1 (p=0.03). The optimal CT-proET-1 cut-off of 74.4 pmol/l had a sensitivity of 74% and a specificity of 74%, and the optimal BNP cut-off of 35.9 pg/ml had a sensitivity of 80% und a specificity of 51% for the prediction of a peak VO₂ <14 ml/kg/min.

### CT-proET and BNP at peak exercise for the prediction of a peak VO₂ <14 ml/kg/min

In Figure 2B, ROC curves for CT-proET-1 and BNP not only at rest but also at peak exercise are displayed. The AUC for CT-proET-1 and BNP measured at peak exercise were 0.76 (95%CI 0.65-0.83; p<0.001) and 0.65 (95%CI 0.54-0.75; p<0.001), respectively. The optimal peak exercise CT-proET-1 cut-off of 74.0 pmol/l had a sensitivity of 80% and a specificity of 67%, and the optimal peak exercise BNP cut-off of 128.2 pg/ml had a sensitivity of 44% and a specificity of 82%. Thus, values measured at peak exercise did not add to the information obtained from resting levels.

### WLₘₐₓ combined with biomarkers for the prediction of a peak VO₂ <14 ml/kg/min

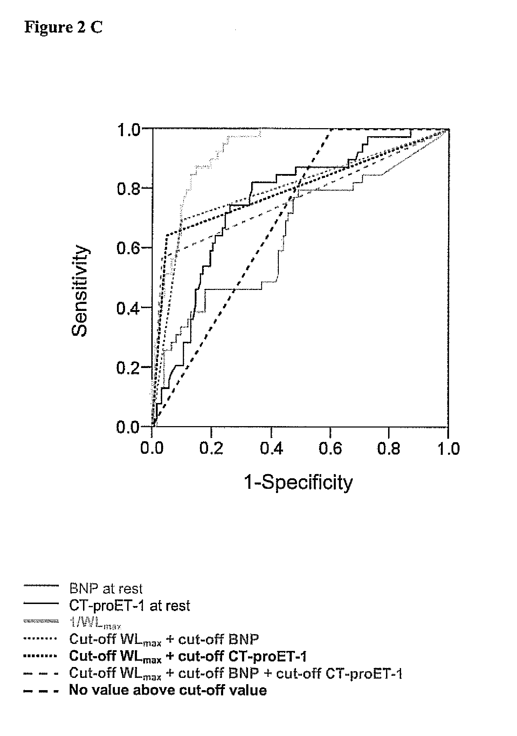

As expected, WLₘₐₓ had a high albeit not perfect accuracy for the prediction of a peak VO₂ <14 ml/kg/min [AUC 0.92 (95%CI 0.88-0.96); p<0.001]. An optimal cut-off of 1.22 Watts/kg had a sensitivity of 87% and a specificity of 85%. As shown in Figure 2C, the AUC for WLₘₐₓ (plotted as 1/Watt per kilogram body weight) was better than that for BNP (p<0.001) and CT-proET-1 (p<0.001). Note that a cut-off of 1.50 Watt/kg was needed to achieve a sensitivity of 100% (specificity 67%), and that a specificity of 100% could be achieved if a WLₘₐₓ cut-off of 0.72 Watt/kg (sensitivity 15%) was selected.

In Table 4, test characteristics of the optimal cut-offs for WLₘₐₓ and the biomarkers as well as their combinations are shown. The added use of CT-proET-1 or/and BNP improved specificity and the positive predictive value of WLₘₐₓ for the prediction of a peak VO₂ <14 ml/kg/min from 85% and 65%, respectively to up to 97% and 85%, respectively. On the other hand, there was no patient without any pathological test value (ie. BNP < 35.9 pg/ml, CT-pro-ET < 74.4 pmol/l, WLₘₐₓ >1.22 Watt/kg) who had a peak VO₂ <14 ml/kg/min, ie. sensitivity and negative predictive value of this combination were 100%, whereas sensitivity and negative predictive value of the optimal WLₘₐₓ cut-off alone were 85% and 95%, respectively.

In Figure 2C, the impact of CT-proET-1 and BNP added to WLₘₐₓ for the prediction a peak VO₂ <14 ml/kg/min is visualized. It becomes obvious that in particular CT-proET-1 or a combination of CT-proET-1 and BNP could improve the sensitivity and specificity of the optimal cut-off for WLₘₐₓ.

**Table 1. Patient characteristics**

| | Peak VO₂ <14 ml/kg/min (n=39) | Peak VO₂ ≥14 ml/kg/min (n=123) | P value |
|---|---|---|---|
| Age (years) | 62±14 | 54±16 | 0.007 |
| Male gender (%) | 20 (51%) | 75 (61%) | 0.28 |
| Body mass index (kg/m²) | 28.5±5.5 | 25.5±4.1 | <0.001 |
| **Medical history** | | | |
| Coronary artery disease | 15 (38%) | 18 (15%) | 0.001 |
| Previous myocardial infarction | 10 (26%) | 11 (9%) | 0.007 |
| Valvular heart disease | 2 (5%) | 4 (3%) | 0.59 |
| Other cardiopathy | 3 (8%) | 10 (8%) | 0.93 |
| Peripheral arterial occlusive disease | 4 (10%) | 1 (1%) | 0.003 |
| Chronic obstructive lung disease | 17 (44%) | 22 (18%) | 0.001 |
| Asthma bronchiale | 3 (8%) | 13 (11%) | 0.60 |
| Other pneumopathy | 9 (23%) | 45 (37%) | 0.12 |
| Pulmonary arterial hypertension | 5 (13%) | 5 (4%) | 0.048 |
| **Left ventricular function (n=82)** | | | |
| Left ventricular ejection fraction (%) | 52 (30-60) n=23 | 60 (55-65) n=49 | 0.001 |
| LV diastolic dysfunction | 13 (57%) n=23 | 27 (55%) n=49 | 0.38 |
| **Renal function** | | | |
| Glomerular filtration rate (ml/min) | 91±42 | 99±34 | 0.26 |
| **Medical therapy** | | | |
| β-blocker | 19 (49%) | 27 (22%) | 0.001 |
| ACEI/ARB | 17 (44%) | 26 (21%) | 0.006 |
| Diuretic | 17 (44%) | 21 (17%) | 0.001 |
| Statin | 16 (41%) | 24 (20%) | 0.007 |
| Aspirin | 14 (36%) | 22 (18%) | 0.018 |
| Bronchodilators | 15 (38%) | 31 (25%) | 0.11 |
| Inhaled corticosteroids | 12(31%) | 28 (23%) | 0.31 |
| Oral corticosteroids | 6 (15%) | 21 (17%) | 0.81 |
| **Symptoms** | | | |
| Dyspnea functional class | | | <0.001 |
| NYHA I | 6 (15%) | 32 (26%) | |
| NYHA II | 10 (26%) | 63 (51%) | |
| NYHA III | 22 (56%) | 25 (20.5%) | |
| NYHA IV | 1 (3%) | 3 (2.5%) | |
| Typical angina | 7 (18%) | 4 (3%) | 0.32 |
| Non-typical angina/chest pain | 3 (8%) | 5 (4%) | 0.36 |

| | | | |
|---|---|---|---|
| Data are given as counts and percentages, mean±standard deviation, or median (interquartile range). ACEI: angiotensin-converting-enyzme inhibitor; ARB: angiotensin receptor blocker. NYHA: New York Heart Association | | | |

**Table 2. Cardiopulmonary exercise testing.**

| | Peak VO₂ <14 ml/kg/min (n=39) | Peak VO₂ ≥14 ml/kg/min (n=123) | P value |
|---|---|---|---|
| **Heart rate and blood pressure response** | | | |
| Resting heart rate (bpm) | 82±15 | 79±14 | 0.30 |
| Peak heart rate (bpm) | 114±18 | 139±27 | <0.001 |
| %predicted heart rate | 71±12 | 84±14 | <0.001 |
| Resting systolic blood pressure (mmHg) | 126±26 | 123±18 | 0.34 |
| Resting diastolic blood pressure (mmHg) | 84±13 | 85±11 | 0.66 |
| Peak systolic blood pressure (mmHg) | 166±31 | 180±32 | 0.019 |
| Peak diastolic blood pressure (mmHg) | 92±17 | 91±15 | 0.77 |
| **Exercise capacity** | | | |
| Exercise time (minutes) | 4.6 (3.7-5.5) | 6.5 (5.3-8.0) | <0.001 |
| Maximal workload (Watt) | 81 (65-92) | 129 (99-166) | <0.001 |
| %predicted maximal workload | 62±23 | 89±25 | <0.001 |
| Body weight-corrected maximal | 0.98 (0.84-1.13) | 1.73 (1.33-2.24) | <0.001 |
| workload (Watt/kg) | | | |
| Peak VO₂ (l/min) | 0.93 (0.76-1.07) | 1.47 (1.16-1.92) | <0.001 |
| %predicted peak VO₂ | 52±14 | 80±18 | <0.001 |
| Body weight-corrected peak VO₂ | 11.7 (10.5-13.1) | 19.8 (16.5-25.0) | <0.001 |
| (ml/kg/min) | | | |
| Respiratory exchange ratio at peak | 1.12 (1.01-1.28) | 1.18 (1.05-1.27) | 0.23 |
| exercise | | | |
| Lactate at rest (mmol/l) | 1.3 (1.1-1.7) | 1.0 (0.7-1.4) | 0.005 |
| Lactate at peak exercise (mmol/l) | 4.2 (3.0-5.3) | 6.3 (4.3-8.5) | <0.001 |
| **Ventilation** | | | |
| FEV₁ (l) | 1.86±0.73 | 2.55±0.83 | <0.001 |
| FEV₁ (% predicted) | 70±20 | 84±20 | <0.001 |
| FEV₁/FVC ratio | 0.71 (0.60-0.74) | 0.73 (0.67-0.79) | 0.044 |
| Peak minute ventilation (l/min) | 45.8±14.7 | 66.2±22.2 | <0.001 |
| Respiratory rate at rest (min⁻¹) | 21 (17-27) | 19 (15-22) | 0.02 |
| Peak respiratory rate (min⁻¹) | 34 (28-39) | 36 (30-41) | 0.14 |
| Breathing reserve (%) | 29±19 | 30±16 | 0.84 |
| **Blood gases** | | | |
| Hemoglobin (g/l) | 13.9±1.9 | 14.4±1.6 | 0.11 |
| Carboxyhemoglobin (%) | 1.5 (1.2-2.7) | 1.5 (1.2-2.1) | 0.94 |
| Oxygen saturation at rest (%) | 95 (93-96) | 95 (94-96) | 0.50 |
| Peak exercise oxygen saturation (%) | 95 (90-96) | 95 (93-97) | 0.90 |
| PaO₂ at rest (mmHg) | 81±17 | 84±13 | 0.42 |
| PaO₂ at peak exercise (mmHg) | 83±24 | 92±17 | 0.011 |
| PaCO₂ at rest (mmHg) | 33±5 | 36±5 | 0.003 |
| PaCO₂ at peak exercise (mmHg) | 34±7 | 35±5 | 0.14 |
| Alveolar-arterial gradient at rest (mmHg) | 29±14 | 20±12 | <0.001 |
| Alveolar-arterial gradient at peak exercise | 33±20 | 23±15 | 0.003 |
| (mmHg) | | | |

| | | | |
|---|---|---|---|
| Data are given as mean±standard deviation or median (interquartile range). FEV₁: forced expiratory volume within the first second; FVC: forced vital capacity; Peak VO₂: peak oxygen consumption; PaO₂: arterial oxygen pressure; PaCO₂: arterial carbon dioxide pressure. | | | |

**Table 3. B-type natriuretic peptide (BNP) and C-terminal-pro-endothelin-1 (CT-proET-1) levels at rest and at peak exercise and absolute (ΔBNP, ΔCT-proET-1) and relative (%ΔBNP, %ΔCT-proET-1) changes from rest to peak exercise.**

| | Peak VO₂ <14 ml/kg/min (n=39) | Peak VO₂ ≥14 ml/kg/min (n=123) | P value |
|---|---|---|---|
| BNP at rest (pg/ml) | 46 (37-270) | 35(15-84) | 0.006 |
| BNP at peak exercise (pg/ml) | 79 (40-270) * | 44 (22-98) * | 0.006 |
| ΔBNP (pg/ml) | 9 (0-41) | 9 (0-21) | 0.51 |
| %ΔBNP | 8 (0-40) | 15 (0-41) | 0.53 |
| CT-proET-1 at rest (pmol/l) | 87.3±25.8 | 66.9±23.9 | <0.001 |
| CT-proBT-1 at peak exercise (pmol/l) | 91.2±30.0 * | 69.0±24.3 * | <0.001 |
| ΔCT-proET-1 (pmol/l) | 3.9±8.9 | 1.8±8.6 | 0.15 |
| %ΔCT-proET-1 | 3.9±9.4 | 2.9±15.3 | 0.70 |

| | | | |
|---|---|---|---|
| Data are given as mean±standard deviation or median (interquartile range). * p<0.05 for comparison with rest | | | |

**Table 4. Test characteristics of optimal cut-offs of B-type natriuretic peptide (BNP), C-terminal-pro-endothelin-1 (CT-pro-ET-1), and maximal body weight-indexed external bicycle workload (WLₘₐₓ) alone or in combination for the prediction of a peak oxygen consumption <14 ml/kg/min.**

| | Sensitivity (%) | Specificity (%) | PPV (%) | NP.V (%) |
|---|---|---|---|---|
| BNP >35.9 pg/ml | 80 | 51 | 34 | 89 |
| CT-proET-1 >74.4 pmol/l | 74 | 74 | 48 | 90 |
| WLₘₐₓ <1.22 Watt/kg | 87 | 85 | 65 | 95 |
| BNP >35.9 pg/ml +CT-proET-1 >74.4 | 64 | 82 | 53 | 88 |
| pmol/l | | | | |
| BNP >35.9 pg/ml + WLₘₐₓ <1.22 | 69 | 90 | 69 | 90 |
| Watt/kg | | | | |
| CT-proET-1 >74.4 pmol/l + WLₘₐₓ | 64 | 95 | 81 | 89 |
| <1.22 Watt/kg | | | | |
| BNP >35.9 pg/ml + CTpro-ET-1 >74.4 | 56 | 97 | 85 | 88 |
| pmol/l + WLₘₐₓ <1.22 Watt/kg | | | | |
| No pathological test value | 100 | 40 | 35 | 100 |

| | | | | |
|---|---|---|---|---|
| PPV: positive predictive value, NPV: negative predictive value. | | | | |

## Claims

1. An in vitro method as a surrogate for the measurement of peak VO₂ for a subject not having heart failure comprising the steps of:
○ determining the level of Pro-Endothelin-1 (ProET-1) or fragments thereof of at least 12 amino acids in a sample obtained from said subject;
○ using said level of Pro-Endothelin-1 (ProET-T) or fragments thereof of at least 12 amino acids as a surrogate marker for peak VO₂.

2. A method according to claim 1, wherein the sample was taken from said subject at rest and thus resting levels of Pro-Endothelin-1 (ProET-1) or fragments thereof of at least 12 amino acids are determined.

3. A method according to claim 1 or 2 for the assessment of severely impaired peak oxygen consumption.

4. A method according to claims 1 to 3, wherein the level of Pro-Endothelin-1 (ProET-1) or fragments thereof of at least 12 amino acids is measured in addition to WLₘₐₓ and wherein the measurement of Pro-Endothelin-1 (ProET-1) or fragments thereof of at least 12 amino acids leads to an improvement in the accuracy of WLₘₐₓ for the prediction of impaired peak VO₂.

5. A method according to claims 1 to 4, wherein the level of proBNP or fragments thereof of at least 12 amino acids including BNP or NT-proBNP is additionally measured.

6. A method according to claims 1 to 5, wherein CT-proET-1 is measured and a threshold value of 74.4 (+/- 20%) pmol/L is used.

7. A method according to claim 5, wherein BNP is measured and a threshold value of 35.9 (+/- 20%) pg/mL is used.

8. A method according to claims 1 to 7, wherein said sample is selected from the group comprising a blood sample, a serum sample, and a plasma sample.

9. A method according to claims 1 to 8, wherein said level of Pro-Endothelin-1 (ProET-1) or fragments thereof of at least 12 amino acids is measured with a sandwich immunoassay.

10. The use of a method according claims 1 to 9 for risk assessment of patients, who are considered candidates for non-cardiac surgery.
